# FASCICULE DE BREVET EUROPEEN

(11) **EP 3 208 599 B1**
(45) Date de publication et mention de la délivrance du brevet: **09.03.2022**
(21) Numéro de dépôt: 17165937.8
(22) Date de dépôt: 21.07.2014
(51) Int. Cl.: G01N 21/45, G01N 21/47, G01N 21/51, G01N 15/14, G01N 33/483, G03H 1/00, G03H 1/04, C12M 1/00, G01N 15/10, G01N 21/03, G01N 21/85

(54) **PROCEDE D'UTILISATION D'UNE SONDE IMMERGEABLE, EN FONCTION D'UNE CONCENTRATION EN PARTICULES**
EINSATZVERFAHREN EINER TAUCHSONDE IN ABHÄNGIGKEIT EINER PARTIKELKONZENTRATION
METHOD FOR USING A SUBMERSIBLE PROBE, AS A FUNCTION OF A PARTICLE CONCENTRATION

(30) Priorité: 23.07.2013 FR 1357240
(43) Date de publication de la demande: 23.08.2017
(62) Demande divisionnaire de: 14747543.8
(73) Titulaire: Commissariat à l'Energie Atomique et aux Energies Alternatives, 75015 Paris (FR)
(72) Inventeur: ALLIER, Cédric, 38000 GRENOBLE (FR)
(74) Mandataire: Brevalex

(56) Documents cités:
- WO-A1-2012/062805
- US-A- 5 418 615
- US-A1- 2005 264 817
- US-A1- 2012 218 551
- US-B1- 6 905 838
- SUNGKYU SEO ET AL: "Lensfree holographic imaging for on-chip cytometry and diagnostics", LAB ON A CHIP, vol. 9, no. 6, 1 janvier 2009 (2009-01-01) , page 777, XP055093847, ISSN: 1473-0197, DOI: 10.1039/B813943A
- SU TING-WEI ET AL: "High-throughput lensfree imaging and characterization of a heterogeneous cell solution on a chip", BIOTECHNOLOGY AND BIOENGINEERING, WILEY & SONS, HOBOKEN, NJ, US, vol. 102, no. 3, 15 février 2009 (2009-02-15), pages 856-868, XP002584870, ISSN: 0006-3592, DOI: 10.1002//BIT.22116 [extrait le 2008-09-08]
- JAMES P. RYLE ET AL: "Multispectral lensless digital in-line holographic microscope: LED illumination", PROCEEDINGS OF SPIE, vol. 7717, 30 avril 2010 (2010-04-30), pages 77170P-77170P-8, XP055095750, ISSN: 0277-786X, DOI: 10.1117/12.854960

## Description

### DOMAINE TECHNIQUE

La présente invention concerne l'utilisation d'une sonde immergeable en fonction d'une concentration en particules.

### ÉTAT DE LA TECHNIQUE ANTÉRIEURE

On connaît dans l'art antérieur différents procédés pour discriminer une cellule vivante d'une cellule morte, ou en d'autres termes une cellule viable d'une cellule non-viable.

On connaît par exemple des procédés de microscopie à fluorescence. Ces procédés utilisent un microscope optique pour visualiser un échantillon, ainsi qu'un marqueur fluorescent qui se fixe préférentiellement sur une catégorie de cellules, parmi les deux catégories cellules vivantes et cellules mortes. Le marqueur fluorescent absorbe un rayonnement incident à une première longueur d'onde, et émet en réponse un rayonnement de fluorescence à une deuxième longueur d'onde. On nomme « cellules marquées » les cellules fixées au marqueur fluorescent. On voit qu'en éclairant un échantillon de cellules marquées avec un rayonnement à la première longueur d'onde, on peut discriminer une cellule vivante d'une cellule morte en identifiant les cellules émettant le rayonnement de fluorescence à la deuxième longueur d'onde. En pratique, on va observer au microscope des cellules présentant une couleur correspondant à la longueur d'onde du rayonnement de fluorescence, et des cellules ne présentant pas cette couleur.

Pour marquer les cellules vivantes, on peut par exemple utiliser la calcéine AM en tant que marqueur fluorescent se fixant uniquement aux cellules vivantes. On connaît également l'iodure de proprium se fixant uniquement aux cellules mortes.

Un inconvénient de ces procédés est qu'ils sont invasifs : l'échantillon de cellules est perturbé par l'ajout du marqueur fluorescent.

Un autre inconvénient de ces procédés est qu'ils sont onéreux : le microscope à fluorescence doit présenter d'excellentes performances optiques basées sur l'utilisation de composants optiques de grande qualité. De plus, leur champ est limité, souvent inférieur à 1 mm², ce qui ne permet pas l'observation simultanée d'un grand nombre de cellules.

Les inventeurs proposent dans la suite une méthode pour discriminer une cellule vivante d'une cellule morte, qui ne présente pas au moins l'un des inconvénients de l'art antérieur.

La présente invention concerne un procédé d'utilisation d'une sonde immergeable, présentant un intérêt notamment dans le cadre de cette méthode pour discriminer une cellule vivante d'une cellule morte.

A cet égard, on connaît l'article de Sungkyu Seo & al., Lab Chip, 2009, 9, pages 777-787, « Lensfree holographie imaging for on-chip cytometry and diagnostics », décrivant une plate-forme d'imagerie sans lentille de reconnaissance de comptage de cellules.

On connaît également le document US 2005/0264817 A1, qui décrit une sonde immergeable permettant de mesurer un taux d'absorption, un taux de diffusion, ou une intensité d'une émission de fluorescence, dans une solution liquide.

### EXPOSÉ DE L'INVENTION

La présente invention est définie par un procédé d'utilisation d'une sonde immergeable, la sonde immergeable comprenant :
- une source lumineuse, agencée pour illuminer un échantillon d'une solution liquide comprenant des particules ;
- un photodétecteur matriciel disposé en face de la source lumineuse, agencé pour acquérir une figure de diffraction globale de l'échantillon, la figure de diffraction globale comprenant plusieurs figures de diffraction élémentaires associées chacune à une particule ;
- un premier logement étanche, recevant la source lumineuse et présentant une première fenêtre transparente entre la source lumineuse et le photodétecteur matriciel ;
- un second logement étanche, recevant le photodétecteur matriciel, et présentant une seconde fenêtre transparente entre la première fenêtre transparente et le photodétecteur matriciel, la distance entre la première fenêtre transparente et la seconde fenêtre transparente étant variable dans l'espace ou variable dans le temps.

Selon ce procédé :
- on immerge la sonde immergeable dans une solution liquide comprenant des particules telles que des cellules, ladite solution liquide occupant l'espace entre la première fenêtre transparente et la seconde fenêtre transparente ;
- on définit une distance entre la première fenêtre transparente et la seconde fenêtre transparente, en fonction de la concentration en particules de la solution liquide et de sorte qu'au moins 30%, voire 50% ou 80% des figures de diffraction élémentaires d'une figure de diffraction globale ne soient pas superposées avec une autre figure de diffraction élémentaire, chaque figure de diffraction élémentaire étant associée à une particule telle qu'une cellule ; et
à l'aide du photodétecteur matriciel, et pour ladite distance ainsi définie, on acquiert une figure de diffraction globale de l'échantillon, où l'échantillon est formé par une portion de ladite solution liquide située entre la première fenêtre transparente et la seconde fenêtre transparente.

D'autres caractéristiques préférées de l'invention sont définies dans les revendications 2 à 10.

### BRÈVE DESCRIPTION DES DESSINS

La présente invention sera mieux comprise à la lecture de la description d'exemples de réalisation donnés à titre purement indicatif et nullement limitatif, en faisant référence aux dessins annexés parmi lesquels :
- la Figure 1 illustre un dispositif connu d'imagerie sans lentille ;
- les Figures 2A et 2B illustrent une figure de diffraction obtenue à l'aide du dispositif de la Figure 1 et pour une cellule vivante ;
- les Figures 3A et 3B illustrent une figure de diffraction obtenue à l'aide du dispositif de la Figure 1 et pour une cellule morte ;
- la Figure 4A illustre un disque central de la figure de diffraction représentée en Figure 2A ;
- la Figure 4B illustre un disque central de la figure de diffraction représentée en Figure 3A ;
- les Figures 4C et 4D illustrent un indicateur numérique d'une méthode pour discriminer une cellule vivante d'une cellule morte ;
- la Figure 5 illustre l'évolution de la figure de diffraction associée à une cellule, lorsque ladite cellule initialement vivante est progressivement rendue non viable jusqu'à ce qu'elle meurt ;
- la Figure 6 illustre de façon schématique un dispositif pour mettre en œuvre la méthode pour discriminer une cellule vivante d'une cellule morte ;
- la Figure 7 illustre de façon schématique un premier mode de réalisation de sonde immergeable utilisée dans le procédé selon l'invention ;
- la Figure 8 illustre de façon schématique un deuxième mode de réalisation de sonde immergeable utilisée dans le procédé selon l'invention ; et
- la Figure 9 illustre de façon schématique un bioréacteur dans lequel on peut immerger une sonde.

### EXPOSÉ DÉTAILLÉ DE MODES DE RÉALISATION PARTICULIERS

On décrit pour commencer une méthode pour discriminer une cellule vivante d'une cellule morte.

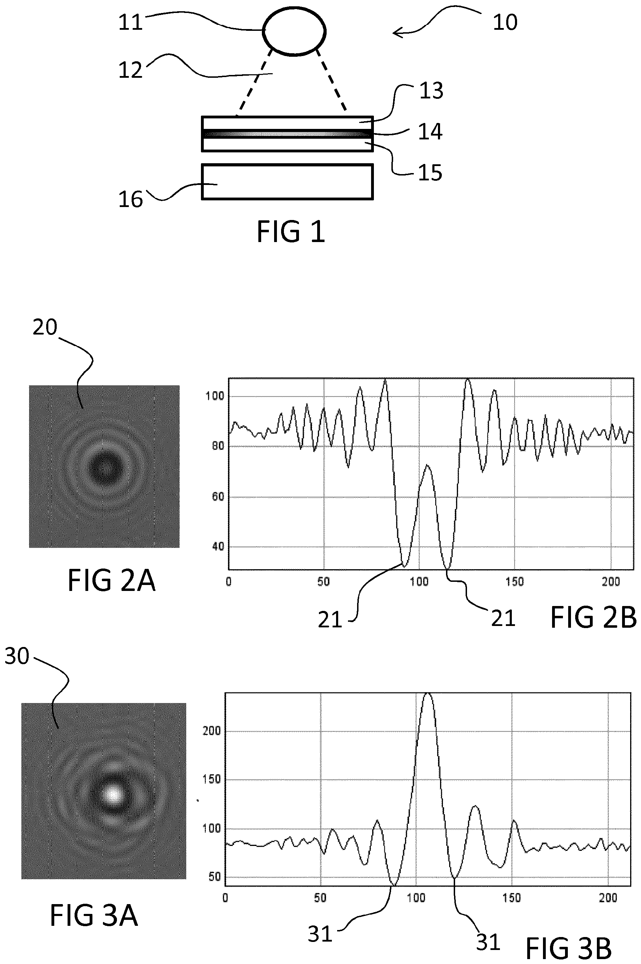

On va tout d'abord décrire, en référence à la Figure 1, un dispositif d'imagerie sans lentille 10. Un tel dispositif est connu de l'homme de l'art, qui saura facilement trouver dans la littérature les détails de réalisation d'un tel dispositif.

Une source lumineuse 11 émet un faisceau lumineux 12 illuminant un échantillon 14. La source lumineuse est, de préférence, cohérente spatialement. La source lumineuse peut être une diode laser, ou une diode électroluminescente (LED) suivie d'un trou de filtrage. Le trou de filtrage permet d'améliorer la cohérence spatiale du faisceau lumineux émis par la LED. Avantageusement, la source lumineuse est également temporellement cohérente.

On utilise par exemple une LED dont le spectre d'émission est centré sur 450 nm, soit une émission dans le bleu. La largeur du pic à 450 nm est de 40 nm. La puissance d'émission de la LED est par exemple comprise entre 100 mW et 1 W. La LED est suivie par un trou de filtrage de diamètre 150 µm, placé directement au contact de la LED.

L'échantillon 14 est placé entre la source lumineuse 11 et un photodétecteur matriciel 16.

Dans l'exemple représenté sur la Figure 1, l'échantillon 14 est un échantillon liquide placé entre deux lamelles 13, 15 transparentes à la longueur d'onde d'émission de la source lumineuse. Le dispositif 10 travaille donc en transmission. La distance entre la lamelle 13 (du côté de la source lumineuse) et la source lumineuse est généralement comprise entre 1 cm et 10 cm.

L'échantillon 14 est par exemple un échantillon d'un liquide biologique tel que le sang, une solution de culture de cellules, un échantillon prélevé dans la nature tel qu'un échantillon de l'eau d'un lac, etc. Le dispositif 10 permet de détecter des particules contenues dans l'échantillon et présentant un faible diamètre, typiquement entre 100 nm et 500 µm.

Le photodétecteur matriciel 16 convertit un rayonnement électromagnétique incident en un signal électrique analogique. Ce photodétecteur matriciel 16 est généralement relié à un convertisseur analogique-numérique de façon à fournir une image numérique. On parle de photodétecteur matriciel, car la surface de détection du photodétecteur est découpée en pixels formant une matrice. Le photodétecteur matriciel 16 est par exemple un capteur CCD (pour l'anglais « *Charge-Coupled Device* ») ou un capteur CMOS (pour l'anglais «*Complementary Metal Oxide Semiconductor* »). Chaque pixel du photodétecteur est par exemple un carré de côté inférieur à 9 µm, et même inférieur à 5 µm, par exemple 2,2 µm. En particulier, on peut utiliser un capteur CMOS de 24 mm², présentant un pas de pixel de 2,2 µm.

Le photodétecteur matriciel 16 détecte une figure de diffraction correspondant à l'influence des particules de l'échantillon 14 sur le faisceau lumineux 12. En particulier, le photodétecteur matriciel 16 détecte une figure de diffraction, correspondant aux interférences entre une onde lumineuse incidente provenant directement de la source lumineuse 11 et une onde lumineuse diffractée par des particules de l'échantillon 14. On nomme « hologramme » une telle figure de diffraction. Un avantage d'une telle figure de diffraction est que le signal détecté est de grande amplitude, grâce à la contribution du signal provenant directement de la source lumineuse. Un autre avantage de cette figure de diffraction est que le champ de vision détecté est large, par exemple supérieur à 20 mm². Le photodétecteur 16 est positionné à proximité de l'échantillon 14, par exemple à 0,8 mm de la lamelle 15 (lamelle du côté du photodétecteur 16).

On détecte donc une figure de diffraction correspondant à un objet, et non directement l'image de cet objet. On nomme cette technique « imagerie sans lentille ». Dans tout le texte, on nomme « dispositif d'imagerie sans lentille » un dispositif de ce type, pour détecter une figure de diffraction correspondant à un objet. Il faut noter qu'un tel dispositif peut comprendre une matrice de microlentilles, servant à focaliser sur chaque pixel le faisceau correspondant aux interférences à détecter. Cependant, un tel dispositif ne comporte pas d'optique de grossissement disposée entre l'objet et le photodétecteur.

L'homme du métier saura aisément positionner les uns par rapport aux autres chacun des éléments parmi la source lumineuse 11, l'échantillon 14 et le photodétecteur 16.

Selon une méthode pour discriminer une cellule vivante d'une cellule morte, on utilise ce dispositif 10 avec un échantillon 14 d'une solution liquide comprenant des cellules. Par exemple, on utilise une solution de cellules cancéreuses HeLa. On choisit en particulier d'utiliser un échantillon volumique : l'épaisseur entre les deux lamelles 13, 15 est par exemple de 0,2 mm. Le volume d'échantillon 14, imagé sur le photodétecteur 16 sous la forme d'une figure de diffraction, est d'environ 5 µL.

On acquiert ainsi, sur le photodétecteur 16, une figure de diffraction globale correspondant aux interférences entre des ondes provenant directement de la source lumineuse 11 et des ondes émises par la source lumineuse puis diffractées par les cellules de l'échantillon 14. La figure de diffraction globale comprend plusieurs figures de diffraction élémentaires. Chaque figure de diffraction élémentaire est associée à une cellule de l'échantillon 14, et correspond aux interférences entre des ondes provenant directement de la source lumineuse 11 et des ondes émises par la source lumineuse puis diffractées par ladite cellule.

On a remarqué qu'une figure de diffraction élémentaire présente un profil caractéristique selon que la cellule est vivante ou morte.

La Figure 2A illustre une figure de diffraction élémentaire 20 associée à une cellule vivante HeLa. La figure de diffraction élémentaire 20 est obtenue à l'aide du dispositif décrit ci-dessus. Sur la Figure 2A, l'axe des abscisses et l'axe des ordonnées forment ensemble le plan du photodétecteur 16. Les teintes foncées correspondent à des zones de faible intensité lumineuse, et les teintes claires correspondent à des zones de forte intensité lumineuse. On observe sur la Figure 2A une alternance d'anneaux sombres et clairs nommés « anneaux de diffraction ». Ces anneaux de diffraction traduisent le profil circulaire des cellules observées. La figure de diffraction élémentaire 20 présente une symétrie axiale autour d'un axe de symétrie passant par le centre commun des anneaux sombres et clairs.

La Figure 2B illustre une coupe de cette figure de diffraction élémentaire 20, dans un plan passant par ledit axe de symétrie. Sur la Figure 2B, l'axe des abscisses correspond à une distance, graduée en pixels. L'axe des ordonnées correspond à une intensité lumineuse, graduée en niveau de gris (de 0 à 255, soient 256 niveaux de gris pour un signal analogique en sortie du photodétecteur 16 converti en signal numérique codé sur 8 bits). Une faible valeur du niveau de gris correspond à une faible intensité lumineuse. Une forte valeur du niveau de gris correspond à une forte intensité lumineuse. On reconnaît sur la Figure 2B l'axe de symétrie de la figure de diffraction élémentaire 20, cet axe étant défini par une droite d'abscisse 105 pixels. Autour de cet axe, on distingue un pic central présentant un maximum dont le niveau de gris vaut environ 70, entouré de deux pics secondaires dont les niveaux de gris valent environ 110. Ce pic central de faible intensité lumineuse se retrouve sur la Figure 2A, sur laquelle on voit que les anneaux sont centrés sur un disque gris.

La Figure 3A illustre une figure de diffraction élémentaire 30 associée à une cellule morte HeLa. La figure de diffraction élémentaire est obtenue à l'aide du dispositif décrit ci-dessus. Là-encore, on observe une alternance d'anneaux sombres et clairs. La figure de diffraction élémentaire 30 présente une symétrie axiale autour d'un axe de symétrie passant par le centre commun des anneaux sombres et clairs.

La Figure 3B illustre une coupe de cette figure de diffraction élémentaire 30, dans un plan passant par ledit axe de symétrie.

Les Figures 3A et 3B correspondent respectivement aux Figures 2A et 2B, mais cette fois-ci dans le cas d'une cellule morte.

On observe sur la Figure 3B l'axe de symétrie de la figure de diffraction élémentaire 30, cet axe étant défini par une droite d'abscisse 105 pixels. Autour de cet axe, on distingue un pic central dont le niveau de gris vaut environ 250, entouré de deux pics secondaires dont les niveaux de gris valent environ 110. Ce pic central de forte intensité lumineuse se retrouve sur la Figure 3A, sur laquelle on voit que les anneaux sont centrés sur un disque blanc.

La différence entre la figure de diffraction élémentaire 20 associée à une cellule vivante et la figure de diffraction élémentaire 30 associée à une cellule morte est suffisamment significative pour permettre une détection de l'état de la cellule à partir de sa figure de diffraction. On va donc pouvoir exploiter cette différence pour discriminer une cellule vivante d'une cellule morte.

On remarque que les deux figures de diffraction élémentaires diffèrent en particulier sur une zone centrale, ici en forme de disque, définie sur une figure de diffraction. Ce disque est centré sur le centre des anneaux de ladite figure de diffraction élémentaire. Il est délimité par le premier anneau d'une figure de diffraction élémentaire. Ce premier anneau correspond ici à l'anneau d'intensité lumineuse minimale. On reconnaît sur la Figure 2B, respectivement 3B, les minima d'intensité 21, respectivement 31, correspondant à ce premier anneau de la figure de diffraction élémentaire. Le disque central est par exemple un disque de moins de 100 pixels de diamètre.

On peut relever qu'une modification du profil d'une cellule, par exemple pour prendre une forme ovale, se traduira par une modification de la figure de diffraction élémentaire associée. La figure de diffraction élémentaire associée présentera toujours une succession de courbes fermées concentriques, que par abus de langage on nommera également « anneaux de diffraction ». Ces courbes fermées entoureront une surface délimitée par un plus petit anneau de diffraction élémentaire.

L'idée à la base de la méthode pour discriminer une cellule vivante d'une cellule morte consiste à utiliser une intensité lumineuse sur une surface centrale d'une figure de diffraction élémentaire, pour classer une cellule correspondante dans la catégorie cellule vivante ou dans la catégorie cellule morte. Cette surface est dite centrale, car elle est concentrique avec les anneaux de diffraction. La surface centrale est notamment inscrite dans une surface délimitée par un plus petit anneau de diffraction. Une telle surface correspond par exemple au disque central tel que défini ci-avant.

On voit donc que l'on offre un procédé pour discriminer une cellule vivante d'une cellule morte, qui ne nécessite pas l'ajout d'une substance de marquage.

Les moyens matériels pour mettre en œuvre ce procédé sont simples et peu onéreux : il s'agit en particulier d'un dispositif d'imagerie sans lentille. Le dispositif d'imagerie ne nécessite pas l'utilisation d'optiques coûteuses. La méthode pour discriminer une cellule vivante d'une cellule morte ne nécessite pas l'utilisation de moyens de calcul très puissants, car on ne cherche pas à reconstruire une image des cellules à partir de leurs figures de diffraction élémentaires. On ne cherche pas non plus à traiter des figures de diffraction élémentaires pour les comparer à une bibliothèque de figures de diffraction de référence. On classe une cellule simplement en utilisant une intensité lumineuse sur une surface prédéterminée.

En outre, puisque l'on ne cherche pas ici à reconstruire l'image d'une cellule, il n'est pas nécessaire de connaître la distance entre la cellule et le photodétecteur. On peut donc facilement analyser une population de cellules contenues dans un volume, et pas seulement dans un plan.

Il n'est pas non plus nécessaire de prévoir une focalisation sur un plan particulier, ce qui permet là-encore d'analyser facilement une population de cellules contenues dans un volume.

Un autre avantage de cette méthode pour discriminer une cellule vivante d'une cellule morte est sa précision : on peut réaliser un comptage précis d'un nombre de cellules vivantes et d'un nombre de cellules mortes, dans une région donnée.

On va maintenant illustrer plus précisément l'utilisation d'une intensité lumineuse sur une surface centrale d'une figure de diffraction élémentaire.

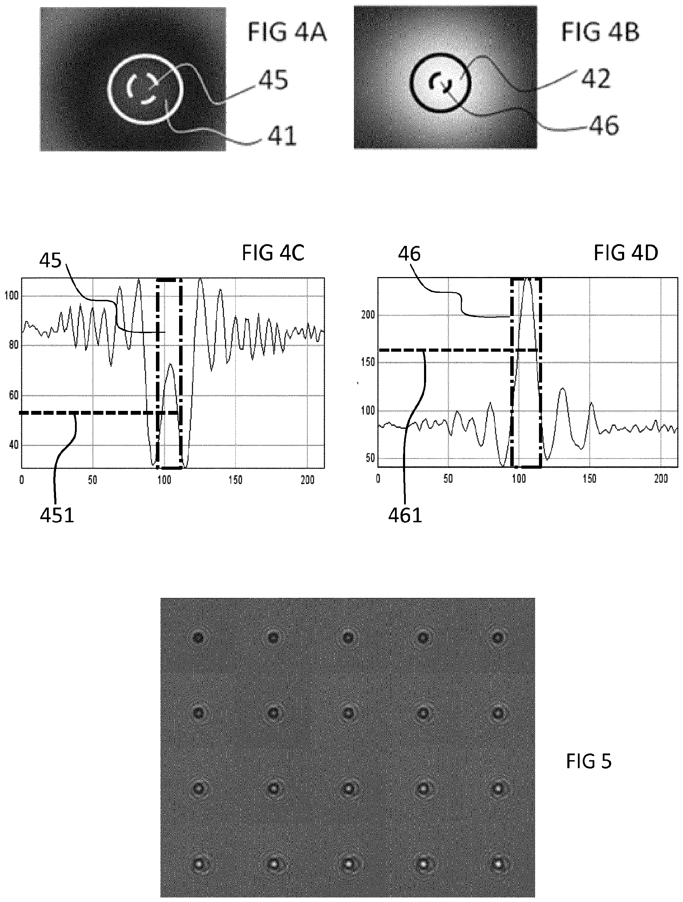

La Figure 4A illustre le disque central 41 tel que défini ci-avant, de la figure de diffraction représentée en Figure 2A. On voit que ce disque central est foncé. Il correspond à de faibles intensités lumineuses, ce qui est caractéristique d'une cellule vivante. Le disque central 41 présente ici un diamètre total d'environ 30 pixels. On peut définir sur le disque central 41 une surface centrale 45, ici un disque concentrique de diamètre inférieur ou égal à celui du disque central 41.

La Figure 4B illustre le disque central 42 tel que défini ci-avant, de la figure de diffraction représentée en Figure 3A. On voit que ce disque central est clair. Il correspond à de fortes intensités lumineuses, ce qui est caractéristique d'une cellule morte. On peut définir sur le disque central 42 une surface centrale 46, ici un disque concentrique de diamètre inférieur ou égal à celui du disque central 42.

On voit donc qu'il suffit d'évaluer l'intensité lumineuse sur une surface centrale inscrite dans le disque central 41 ou 42, pour savoir si une cellule correspondante est vivante ou morte.

On pourra définir un indicateur numérique, servant à caractériser l'intensité lumineuse dans la surface centrale 45 ou 46. Dans l'exemple représenté sur les Figures 4C et 4D, cet indicateur numérique est la valeur moyenne du niveau de gris dans la surface centrale. Puisque la figure de diffraction élémentaire présente ici une symétrie axiale, on peut travailler sur une coupe de cette figure de diffraction passant par l'axe de symétrie. La Figure 4C correspond à la Figure 2B, sur laquelle on a identifié une surface centrale 45. L'indicateur numérique est identifié à la Figure 4C par la droite 451 d'ordonnée 55.

La Figure 4D correspond à la Figure 3B, sur laquelle on a identifié une surface centrale 46. L'indicateur numérique est identifié à la Figure 4D par la droite d'ordonnée 160.

On utilise la valeur de cet indicateur numérique pour classer une cellule dans la catégorie cellule vivante ou cellule morte.

On fixe une valeur de seuil prédéterminée, séparant des valeurs d'indicateur numérique associées à une cellule vivante et des valeurs d'indicateur numérique associées à une cellule morte.

Ainsi, il suffit, pour distinguer une cellule vivante d'une cellule morte, de :
- calculer ledit indicateur numérique ; puis
- comparer l'indicateur numérique à une valeur seuil prédéterminée.

On pourra prévoir différentes types d'indicateurs numériques. Par exemple, on pourra considérer un maximum absolu d'intensité lumineuse, une amplitude crête à crête de l'intensité lumineuse, etc.

On a pris ici l'exemple des cellules HeLa, mais on pourra ainsi classer tout type de cellule.

Le profil caractéristique de la figure de diffraction élémentaire associée à une cellule vivante ou une cellule morte peut dépendre du type de cellule étudié. Ainsi, il pourra arriver qu'une faible intensité lumineuse au centre de la figure de diffraction élémentaire corresponde à une cellule morte et qu'une forte intensité lumineuse au centre de la figure de diffraction élémentaire corresponde à une cellule vivante.

La Figure 5 illustre l'évolution de la figure de diffraction élémentaire associée à une cellule, lorsque ladite cellule initialement vivante est progressivement détériorée jusqu'à ce qu'elle meurt. La cellule HeLa, initialement vivante, est soumise à une température passant de 37°C à 45°C en dix minutes. On a relevé toutes les 30 secondes une figure de diffraction élémentaire de la cellule. Les figures de diffraction élémentaires sont rangées dans l'ordre chronologique de leurs acquisitions, de gauche à droite et de bas en haut. La Figure 5 confirme bien que l'on peut définir un seuil d'intensité sur une surface centrale d'une figure de diffraction élémentaire associée à une cellule, pour discriminer une cellule vivante d'une cellule morte.

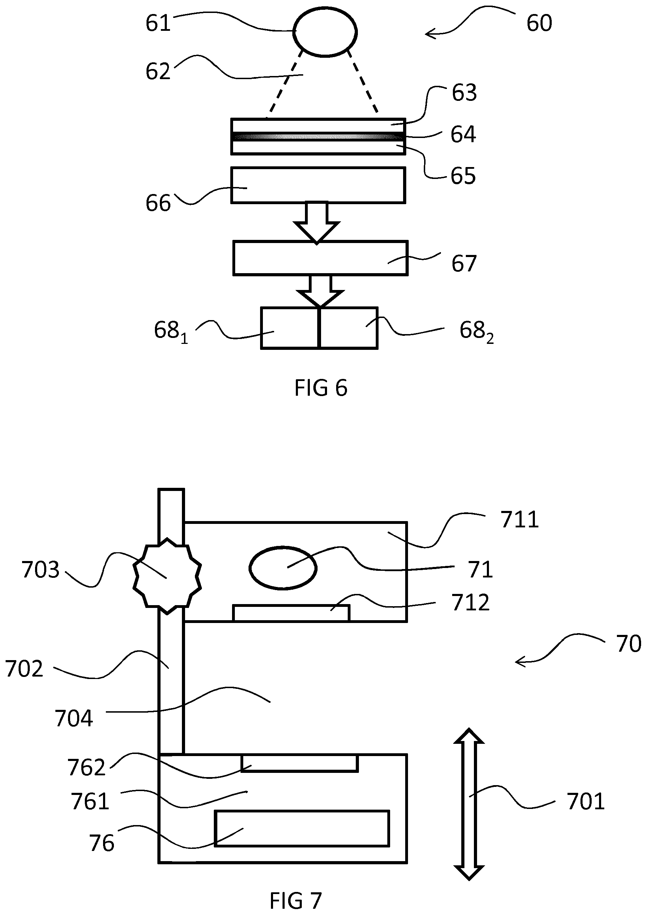

La Figure 6 illustre de façon schématique un dispositif 60 pour mettre en œuvre la méthode pour discriminer une cellule vivante d'une cellule morte.. Sur la Figure 6, les références numériques 61, 62, 63, 64, 65, 66 correspondent respectivement aux références numériques 11, 12, 13, 14, 15 et 16 de la Figure 1.

La source lumineuse 61 est agencée pour illuminer un échantillon d'une solution liquide comprenant des cellules.

Le photodétecteur 66 est un photodétecteur matriciel disposé en face de la source lumineuse, et agencé pour acquérir une figure de diffraction globale de l'échantillon, la figure de diffraction globale comprenant plusieurs figures de diffraction élémentaires associées chacune à une cellule.

Le photodétecteur 66 est relié à des moyens de calcul 67 recevant en entrée la figure de diffraction globale telle que définie ci-avant.

Les moyens de calcul déterminent l'intensité lumineuse sur une surface centrale d'une figure de diffraction élémentaire associée à une cellule, et fournissent en sortie un classement de ladite cellule dans l'une parmi les deux catégories cellule vivante 68₁ ou cellule morte 68₂. La surface centrale est définie précédemment.

En particulier, les moyens de calcul sont agencés pour :
- calculer l'indicateur numérique tel que défini précédemment ;
- comparer cet indicateur numérique à une valeur de seuil prédéterminée ;
- attribuer la cellule correspondant à ladite figure de diffraction élémentaire, à l'une parmi les deux catégories 68₁ et 68₂.

Les moyens de calcul comprennent notamment des moyens électroniques et des moyens informatiques et/ou logiciels. Il s'agit typiquement d'un circuit électronique numérique ou analogique, de préférence dédié, associé à un microprocesseur et/ou un ordinateur.

Cette méthode pour discriminer une cellule vivante d'une cellule morte peut avantageusement être mise en œuvre pour contrôler *in situ* un traitement cellulaire dans un bioréacteur.

La méthode pour discriminer une cellule vivante d'une cellule morte utilise des figures de diffraction élémentaires associées chacune à une cellule. Pour que chaque figure de diffraction élémentaire soit exploitable, il faut que les figures ne soient pas superposées les unes aux autres. Pour une épaisseur donnée, l'échantillon 14 doit donc présenter une concentration en cellules inférieure à une concentration maximale prédéterminée.

Par exemple, dans le mode de réalisation décrit en référence à la Figure 1, on ne peut imager que jusqu'à 10000 figures de diffraction élémentaires sur le photodétecteur 16 présentant une surface de 24 mm². Cela correspond à une concentration maximale de 2^{∗}10⁶ cellules/mL, le volume de l'échantillon 14 étant approximativement de 5 µL.

On voit donc qu'il peut être nécessaire de pouvoir adapter à la concentration en cellules de l'échantillon 14, un dispositif d'imagerie sans lentille utilisé pour mettre en œuvre la méthode pour discriminer une cellule vivante d'une cellule morte.

On propose à cette fin une sonde immergeable, destinée notamment à la mise en œuvre de cette méthode pour discriminer une cellule vivante d'une cellule morte. On pourra également envisager d'utiliser cette sonde immergeable pour d'autres utilisations. Pour cette raison, on a choisi dans la suite de décrire la sonde immergeable seule, indépendamment de moyens de calcul pour mettre en œuvre des étapes de la méthode pour discriminer une cellule vivante d'une cellule morte.

Le principe mis en œuvre dans cette sonde immergeable consiste à adapter l'épaisseur de l'échantillon à sa concentration en particules, ici des cellules.

La sonde immergeable est adaptée à des mesures réalisées en plongeant la sonde dans une solution liquide. On s'affranchit ainsi d'une étape de prélèvement d'échantillon et dépôt sur une lamelle. On limite également les perturbations de l'échantillon dues à son observation, puisque l'échantillon n'est pas isolé du reste de la solution liquide.

La Figure 7 illustre de façon schématique un premier mode de réalisation de sonde immergeable 70, permettant d'adapter l'épaisseur de l'échantillon à sa concentration en particules.

Les références 71 et 76 de la Figure 7 correspondent respectivement aux références 11 et 16 de la Figure 1. La source lumineuse 71 et le photodétecteur 76 forment donc ensemble un dispositif d'imagerie sans lentille tel que décrit en référence à la Figure 1.

La sonde immergeable 70 comprend un premier logement étanche 711, recevant la source lumineuse 71. La source lumineuse 71 est donc protégée à l'intérieur d'un logement étanche, ce qui permet d'immerger entièrement la source, sans risquer de l'endommager. Le premier logement étanche 711 présente une première fenêtre 712, transparente à la longueur d'onde d'émission de la source lumineuse 71. La première fenêtre 712 est située entre la source lumineuse 71 et le photodétecteur 76.

La sonde immergeable 70 comprend également un second logement étanche 761, recevant le photodétecteur 76. Le photodétecteur 76 est donc protégé à l'intérieur d'un logement étanche, ce qui permet de l'immerger entièrement sans risquer de l'endommager. Il ne s'agit pas simplement ici de recouvrir le photodétecteur d'une lamelle de protection, mais de l'envelopper entièrement à l'intérieur d'un boîtier. Le second logement étanche 761 présente une seconde fenêtre 762, transparente à la longueur d'onde d'émission de la source lumineuse 71. La seconde fenêtre 762 est située entre la première fenêtre 712 et le photodétecteur 76.

L'échantillon est formé par une portion de la solution liquide dans laquelle la sonde immergeable 70 est entièrement plongée, cette portion de solution liquide occupant l'espace 704 entre la première fenêtre transparente 712 et la seconde fenêtre transparente 762. La solution liquide comprend des particules telles que des cellules.

Le photodétecteur 76 est agencé pour acquérir une figure de diffraction globale comprenant une pluralité de figures de diffraction élémentaires, chacune associée à une particule. On souhaite adapter l'épaisseur de l'échantillon à sa concentration en cellules, de sorte qu'au moins 80% des figures de diffraction élémentaires d'une figure de diffraction globale ne soient pas superposées avec une autre figure de diffraction élémentaire. Pour cela, la distance entre la première fenêtre transparente 712 et la seconde fenêtre transparente 762 est variable.

Dans le mode de réalisation représenté sur la Figure 7, cette distance est variable dans le temps.

La première fenêtre transparente 712 est parallèle à la seconde fenêtre transparente 762. La seconde fenêtre transparente 762 est mobile selon un axe 701 orthogonal au plan commun des première et seconde fenêtres.

Le second logement 761 est fixe relativement à une tige 702 orientée selon l'axe 701. La tige 702 est crantée. Elle forme une crémaillère, entraînée en translation par un pignon (non représenté). Le pignon est mis en rotation par l'intermédiaire d'une molette 703. La tige 702, entraînant avec elle le second logement 761, peut donc être translatée selon l'axe 701 relativement au premier logement 711.

L'homme du métier pourra prévoir différentes variantes permettant de réaliser un déplacement d'une fenêtre transparente relativement à l'autre fenêtre transparente, selon un axe orthogonal au plan de ces deux fenêtres, sans sortir du cadre de la présente invention.

On peut ainsi ajuster la distance entre la première fenêtre transparente 712 et la seconde fenêtre transparente 762, en déplaçant la seconde fenêtre transparente 762 relativement à la première fenêtre transparente 712.

La sonde immergeable peut être reliée à des moyens de calcul (non représentés) tels que décrits ci-avant en référence à la Figure 6. En d'autres termes, le dispositif 60 tel que représenté à la Figure 6 peut comprendre une sonde immergeable permettant d'adapter l'épaisseur de l'échantillon à sa concentration en particules, la source lumineuse de la source immergeable formant la source lumineuse 61 et le photodétecteur de la sonde immergeable formant le photodétecteur 66.

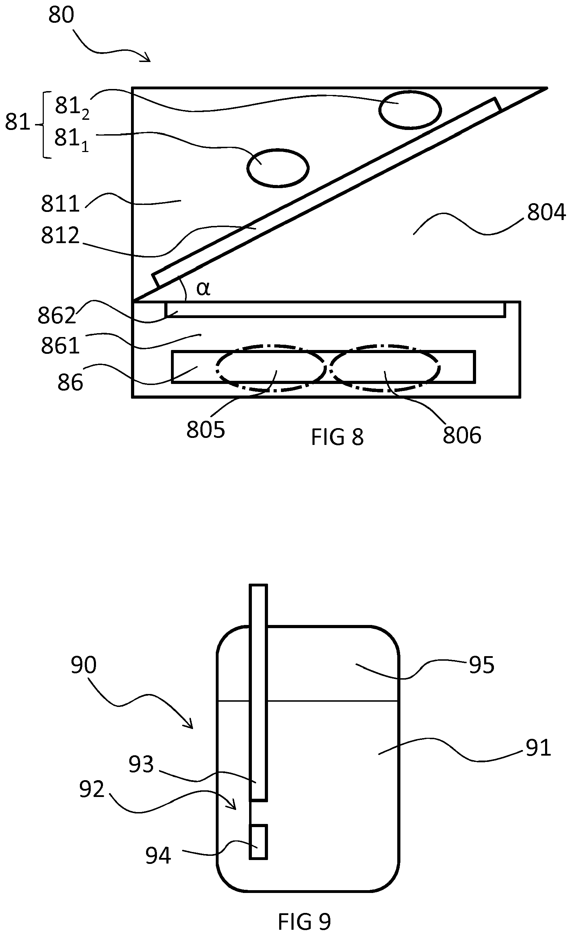

La Figure 8 illustre de façon schématique un deuxième mode de réalisation de sonde immergeable 80 permettant d'adapter l'épaisseur de l'échantillon à sa concentration en particules.

La sonde immergeable 80 ne sera décrite que pour ses différences relativement à la sonde immergeable 70. Sur la Figure 8, les références numériques 81, 811, 812, 86, 861, 862 et 804 correspondent respectivement aux références numériques 71, 711, 712, 76, 761, 762 et 704 de la Figure 7.

Dans le mode de réalisation représenté sur la Figure 8, la distance entre la première fenêtre transparente 812 et la seconde fenêtre transparente 862 est variable dans l'espace.

La première fenêtre transparente 812 est inclinée d'un angle α relativement à la seconde fenêtre transparente 862, ces deux fenêtres étant planes. L'angle α est typiquement compris entre 10° et 45°.

En variante, on pourrait prévoir une fenêtre plane, et une fenêtre présentant un profil en marches d'escalier.

La source lumineuse 81 est formée par plusieurs sources lumineuses unitaires 81₁, 81₂ réparties au-dessus de la première fenêtre transparente 812. Cette caractéristique n'est pas fondamentale, et on pourra également prévoir d'utiliser une source lumineuse faite d'une unique LED ou diode laser, comme décrit en référence à la Figure 1.

Une surface 805 du photodétecteur 86 correspond à une faible distance entre la première fenêtre transparente 812 et la seconde fenêtre transparente 862. On utilise cette surface 805 pour acquérir une figure de diffraction globale lorsque la solution liquide présente par exemple une forte concentration, par exemple supérieure à 10⁶ cellules/mL. La surface 805 est située en face de la source lumineuse unitaire 81₁.

Une surface 806 du photodétecteur 86 correspond à une grande distance entre la première fenêtre transparente 812 et la seconde fenêtre transparente 862. On utilise cette surface 806 pour acquérir une figure de diffraction globale lorsque la solution liquide présente une faible concentration, par exemple comprise entre 10⁴ et 10⁵ cellules/mL. La surface 806 est située en face de la source lumineuse unitaire 81₂.

On peut ainsi ajuster la distance entre la première fenêtre transparente 812 et la seconde fenêtre transparente 862, en définissant une portion du photodétecteur 86 utilisée pour obtenir des figures de diffraction élémentaires à analyser.

La Figure 9 illustre de façon schématique un bioréacteur 90. Le bioréacteur 90 désigne ici un dispositif destiné à cultiver des cellules ou des tissus dans le cadre d'une culture cellulaire, ou à mettre en œuvre un processus chimique impliquant des organismes ou des substances biochimiques actives dérivées de ces organismes.

Un bioréacteur est conçu pour offrir des conditions optimales à une culture cellulaire ou un processus chimique. Pour cela, on ajuste et contrôle différents paramètres tels que :
- l'agitation de la solution liquide contenant les cellules, organismes ou substances biochimiques actives ;
- le pH de cette solution ;
- la température de cette solution ;
- le taux d'oxygène dissout dans cette solution.

Dans l'exemple représenté sur la Figure 9, le bioréacteur 90 reçoit une solution liquide 91 comprenant des cellules. Cette solution est située dans une cuve 95 du bioréacteur.

Des ouvertures pratiquées dans la cuve du bioréacteur permettent d'insérer des sondes pour contrôler les conditions à l'intérieur du bioréacteur 90. L'une de ces ouvertures est utilisée pour insérer une sonde immergeable 92 permettant d'adapter l'épaisseur de l'échantillon à sa concentration en particules. La sonde immergeable 92 présente une forme de cylindre allongé, de diamètre inférieur à 12 mm et de longueur supérieure à 50 mm. La sonde immergeable 92 présente une partie supérieure 93 recevant le premier logement étanche tel que décrit ci-avant, et une partie inférieure 94 recevant le second logement étanche tel que décrit ci-avant. L'espace entre ces deux logements est occupé par la solution liquide 91.

On peut ainsi mesurer l'évolution au cours du temps d'un taux de cellules vivantes dans le bioréacteur. Le temps d'acquisition d'une figure de diffraction globale est avantageusement de l'ordre de la milliseconde, pour éviter l'effet du mouvement des cellules.

## Revendications

1. Procédé d'utilisation d'une sonde immergeable (70 ; 80; 92), la sonde immergeable comprenant :
- une source lumineuse (71; 81), agencée pour illuminer un échantillon d'une solution liquide comprenant des particules;
- un photodétecteur matriciel (76 ; 86) disposé en face de la source lumineuse, agencé pour acquérir une figure de diffraction globale de l'échantillon, la figure de diffraction globale comprenant plusieurs figures de diffraction élémentaires (20 ; 30) associées chacune à une particule ;
- un premier logement étanche (711; 811), recevant la source lumineuse et présentant une première fenêtre transparente (712 ; 812) entre la source lumineuse et le photodétecteur matriciel ;
- un second logement étanche (761; 861), recevant le photodétecteur matriciel, et présentant une seconde fenêtre transparente (762 ; 862) entre la première fenêtre transparente et le photodétecteur matriciel, la distance entre la première fenêtre transparente et la seconde fenêtre transparente étant variable dans l'espace ou variable dans le temps ; dans lequel le procédé comprend les étapes suivantes :
- on immerge la sonde immergeable (70; 80; 92) dans une solution liquide comprenant des particules telles que des cellules, ladite solution liquide occupant l'espace entre la première fenêtre transparente (712 ; 812) et la seconde fenêtre transparente (762 ; 862) ;
- on définit une distance entre la première fenêtre transparente (712 ; 812) et la seconde fenêtre transparente (762 ; 862), en fonction de la concentration en particules de la solution liquide et de sorte qu'au moins 30% des figures de diffraction élémentaires (20; 30) d'une figure de diffraction globale ne soient pas superposées avec une autre figure de diffraction élémentaire, chaque figure de diffraction élémentaire étant associée à une particule telle qu'une cellule ; et
à l'aide du photodétecteur matriciel (76 ; 86), et pour ladite distance ainsi définie, on acquiert une figure de diffraction globale de l'échantillon, où l'échantillon est formé par une portion de ladite solution liquide située entre la première fenêtre transparente (712 ; 812) et la seconde fenêtre transparente (762 ; 862).

2. Procédé selon la revendication 1, dans lequel on définit la distance entre la première fenêtre transparente (712 ; 812) et la seconde fenêtre transparente (762 ; 862), de sorte qu'au moins 50% des figures de diffraction élémentaires (20 ; 30) d'une figure de diffraction globale ne soient pas superposées avec une autre figure de diffraction élémentaire.

3. Procédé selon la revendication 1, dans lequel on définit la distance entre la première fenêtre transparente (712 ; 812) et la seconde fenêtre transparente (762 ; 862), de sorte qu'au moins 80% des figures de diffraction élémentaires (20 ; 30) d'une figure de diffraction globale ne soient pas superposées avec une autre figure de diffraction élémentaire.

4. Procédé selon l'une quelconque des revendications 1 à 3, dans lequel la distance entre la première fenêtre transparente (712 ; 812) et la seconde fenêtre transparente (762 ; 862) dépend d'une concentration en particules dans la solution liquide.

5. Procédé selon l'une quelconque des revendications 1 à 4, dans lequel
- la distance entre la première fenêtre transparente (812) et la seconde fenêtre transparente (862) est variable dans l'espace, avec la première fenêtre transparente et la deuxième fenêtre transparente qui définissent deux plans inclinés l'un relativement à l'autre, de sorte que la distance entre la première fenêtre transparente et la seconde fenêtre transparente dépend de l'emplacement sur la seconde fenêtre transparente (862) ;
- on définit la distance entre la première fenêtre transparente (812) et la seconde fenêtre transparente (862) en définissant une portion (805 ; 806) du photodétecteur matriciel (86) utilisée pour obtenir la figure de diffraction globale ; et
- on acquiert ladite figure de diffraction globale à l'aide de ladite portion (805 ; 806) du photodétecteur matriciel (86).

6. Procédé selon l'une quelconque des revendications 1 à 4, dans lequel :
- la distance entre la première fenêtre transparente (712) et la seconde fenêtre transparente (762) est variable dans le temps, avec la première fenêtre transparente et la deuxième fenêtre transparente qui définissent deux plans parallèles entre eux, et avec la sonde immergeable qui comprend des moyens (702, 703) pour déplacer la seconde fenêtre transparente relativement à la première fenêtre transparente ; et
- on déplace la première fenêtre transparente relativement à la deuxième fenêtre transparente ; et
- on acquiert ladite figure de diffraction globale à l'aide du photodétecteur matriciel (76).

7. Procédé selon la revendication 6, dans lequel les moyens (702, 703) pour déplacer la seconde fenêtre transparente relativement à la première fenêtre transparente comprennent une crémaillère entraînée en translation par un pignon, et fixe relativement au second logement étanche (761).

8. Procédé selon l'une quelconque des revendications 1 à 7, dans lequel la source lumineuse (71 ; 81) est spatialement cohérente.

9. Procédé selon l'une quelconque des revendications 1 à 8, dans lequel la sonde immergeable (92) est immergée dans une solution liquide (91), ladite solution liquide (91) étant située dans une cuve (95) d'un bioréacteur (90).

10. Procédé selon l'une quelconque des revendications 1 à 9, dans lequel le procédé il comprend ensuite les étapes suivantes :
- on acquiert, à l'aide du photodétecteur matriciel (76 ; 86), une figure de diffraction globale, la figure de diffraction globale comprenant une pluralité de figures de diffraction élémentaires (20 ; 30) associées chacune à une cellule, chaque figure de diffraction élémentaire présentant une succession de courbes fermées concentriques ; et
- on classe une cellule dans l'une parmi les deux catégories cellule vivante (68₁) ou cellule morte (68₂), en fonction d'une intensité lumineuse sur une surface centrale (45 ; 46) d'une figure de diffraction élémentaire (20 ; 30) associée à ladite cellule.

## Patentansprüche

1. Verfahren zur Verwendung einer Tauchsonde (70; 80; 92), wobei die Tauchsonde umfasst:
- eine Lichtquelle (71; 81), die dazu angeordnet wird, eine Probe einer Partikel enthaltenden flüssigen Lösung zu beleuchten;
- einen Matrixphotodetektor (76; 86), der gegenüber der Lichtquelle platziert und dazu angeordnet wird, ein globales Beugungsmuster der Probe zu erfassen, wobei das globale Beugungsmuster mehrere elementare Beugungsmuster (20; 30) umfasst, die jeweils einem Partikel zugeordnet werden;
- ein erstes dichtes Gehäuse (711; 811), das die Lichtquelle aufnimmt und ein erstes transparentes Fenster (712; 812) zwischen der Lichtquelle und dem Matrixphotodetektor aufweist;
- ein zweites dichtes Gehäuse (761; 861), das den Matrixphotodetektor aufnimmt und ein zweites transparentes Fenster (762; 862) zwischen dem ersten transparenten Fenster und dem Matrixphotodetektor aufweist, wobei der Abstand zwischen dem ersten transparenten Fenster und dem zweiten transparenten Fenster räumlich variabel oder zeitlich variabel ist;
wobei das Verfahren die folgenden Schritte umfasst:
- Eintauchen der Tauchsonde (70; 80; 92) in eine flüssige Lösung, die Partikel wie Zellen enthält, wobei die flüssige Lösung den Raum zwischen dem ersten transparenten Fenster (712; 812) und dem zweiten transparenten Fenster (762; 862) einnimmt;
- Definieren eines Abstands zwischen dem ersten transparenten Fenster (712; 812) und dem zweiten transparenten Fenster (762; 862) in Abhängigkeit von der Partikelkonzentration der flüssigen Lösung derart, dass wenigstens 30% der elementaren Beugungsmuster (20; 30) eines globalen Beugungsmusters nicht mit einem anderen elementaren Beugungsmuster überlagert werden, wobei jedes elementare Beugungsmuster einem Partikel, wie z.B. einer Zelle, zugeordnet wird;
- und wobei mit Hilfe des Matrixphotodetektors (76; 86) bei dem so definierten Abstand ein globales Beugungsmuster der Probe erfasst wird, wobei die Probe durch einen Teil der flüssigen Lösung gebildet wird, der sich zwischen dem ersten transparenten Fenster (712; 812) und dem zweiten transparenten Fenster (762; 862) befindet.

2. Verfahren nach Anspruch 1, wobei der Abstand zwischen dem ersten transparenten Fenster (712; 812) und dem zweiten transparenten Fenster (762; 862) derart festgelegt wird, dass wenigstens 50% der elementaren Beugungsmuster (20; 30) eines globalen Beugungsmusters nicht mit einem anderen elementaren Beugungsmuster überlappt werden.

3. Verfahren nach Anspruch 1, wobei der Abstand zwischen dem ersten transparenten Fenster (712; 812) und dem zweiten transparenten Fenster (762; 862) so festgelegt wird, dass wenigstens 80% der elementaren Beugungsmuster (20; 30) eines globalen Beugungsmusters nicht mit einem anderen elementaren Beugungsmuster überlagert werden.

4. Verfahren nach einem der Ansprüche 1 bis 3, wobei der Abstand zwischen dem ersten transparenten Fenster (712; 812) und dem zweiten transparenten Fenster (762; 862) von einer Partikelkonzentration in der flüssigen Lösung abhängt.

5. Verfahren nach einem der Ansprüche 1 bis 4, wobei
- der Abstand zwischen dem ersten transparenten Fenster (812) und dem zweiten transparenten Fenster (862) räumlich variabel ist, wobei das erste transparente Fenster und das zweite transparente Fenster zwei relativ zueinander geneigte Ebenen definieren, so dass der Abstand zwischen dem ersten transparenten Fenster und dem zweiten transparenten Fenster von der Position auf dem zweiten transparenten Fenster (862) abhängt;
- Definieren des Abstands zwischen dem ersten transparenten Fenster (812) und dem zweiten transparenten Fenster (862) durch Definieren eines Abschnitts (805; 806) des Matrixphotodetektors (86), der zum Erhalten des globalen Beugungsmusters verwendet wird; und
- Erfassen des globalen Beugungsmusters unter Verwendung des Abschnitts (805; 806) des Matrixphotodetektors (86).

6. Verfahren nach einem der Ansprüche 1 bis 4, wobei:
- der Abstand zwischen dem ersten transparenten Fenster (712) und dem zweiten transparenten Fenster (762) zeitlich variabel ist, wobei das erste transparente Fenster und das zweite transparente Fenster zwei zueinander parallele Ebenen definieren, und wobei die Tauchsonde Mittel (702, 703) zum Bewegen des zweiten transparenten Fensters relativ zum ersten transparenten Fenster aufweist; und
- Bewegen des ersten transparenten Fensters relativ zum zweiten transparenten Fenster; und
- Erfassen des globalen Beugungsmusters mittels des Matrixphotodetektors (76).

7. Verfahren nach Anspruch 6, wobei die Mittel (702, 703) zum Bewegen des zweiten transparenten Fensters relativ zum ersten transparenten Fenster einen Zahnkranz umfassen, der durch ein Ritzel translatorisch angetrieben wird und relativ zum zweiten abgedichteten Gehäuse (761) fixiert ist.

8. Verfahren nach einem der Ansprüche 1 bis 7, wobei die Lichtquelle (71; 81) räumlich kohärent ist.

9. Verfahren nach einem der Ansprüche 1 bis 8, wobei die Tauchsonde (92) in eine flüssige Lösung (91) eingetaucht wird, wobei sich die flüssige Lösung (91) in einem Tank (95) eines Bioreaktors (90) befindet.

10. Verfahren nach einem der Ansprüche 1 bis 9, wobei das Verfahren ferner die folgenden Schritte umfasst:
- Erfassen eines globalen Beugungsmusters mittels des Matrixphotodetektors (76; 86), wobei das globale Beugungsmuster eine Vielzahl von elementaren Beugungsmustern (20; 30) umfasst, die jeweils einer Zelle zugeordnet werden, wobei jedes elementare Beugungsmuster eine Folge von konzentrischen geschlossenen Kurven aufweist; und
- Einordnen einer Zelle in eine der beiden Kategorien lebende Zelle (68₁) oder tote Zelle (68₂) auf der Grundlage einer Lichtintensität auf einer zentralen Fläche (45; 46) eines elementaren Beugungsmusters (20; 30), das der Zelle zugeordnet wird.

## Claims

1. Method of using an immersible probe (70; 80; 92), the immersible probe comprising:
- a light source (71; 81), arranged to illuminate a sample of a liquid solution containing particles;
- a matrix photodetector (76; 86) disposed facing the light source, arranged to acquire a global diffraction figure of the sample, the global diffraction figure comprising a plurality of elementary diffraction figures (20; 30) each associated with a particle;
- a first tight housing (711; 811), holding the light source and having a first transparent window (712; 812) between the light source and the matrix photodetector;
- a second tight housing (761; 861), holding the matrix photodetector, and having a second transparent window (762; 862) between the first transparent window and the matrix photodetector, the distance between the first transparent window and the second transparent window being variable in space or variable in time;
in which the method comprises the following steps:
- the immersible probe (70; 80; 92) is immersed in a liquid solution comprising particles such as cells, said liquid solution occupying the space between the first transparent window (712; 812) and the second transparent window (762; 862);
- a distance between the first transparent window (712; 812) and the second transparent window (762; 862) is defined as a function of the particle concentration in the liquid solution and such that at least 30% of the elementary diffraction figures (20; 30) in a global diffraction figure are not superimposed with another elementary diffraction figure, each elementary diffraction figure being associated with a particle such as a cell; and
with the use of the matrix photodetector (76; 86), and for said distance thus defined, a global diffraction figure of the sample is acquired, wherein the sample is formed by a portion of said liquid solution disposed between the first transparent window (712; 812) and the second transparent window (762; 862).

2. Method according to claim 1, wherein the distance between the first transparent window (712; 812) and the second transparent window (762; 862) is defined such that at least 50% of the elementary diffraction figures (20; 30) of a global diffraction figure are not superimposed with another elementary diffraction figure.

3. Method according to claim 1, wherein the distance between the first transparent window (712; 812) and the second transparent window (762; 862) is defined such that at least 80% of the elementary diffraction figures (20; 30) of a global diffraction figure are not superimposed with another elementary diffraction figure.

4. Method according to any one of claims 1 to 3, wherein the distance between the first transparent window (712; 812) and the second transparent window (762; 862) depends on a particles concentration in the liquid solution.

5. Method according to any one of claims 1 to 4, wherein:
- the distance between the first transparent window (812) and the second transparent window (862) is variable in space, with the first transparent window and the second transparent window defining two planes inclined relative to each other, such that the distance between the first transparent window and the second transparent window depends on the location on the second transparent window (862);
- the distance between the first transparent window (812) and the second transparent window (862) is defined by defining a portion (805; 806) of the matrix photodetector (86) used to obtain the global diffraction figure; and
- said global diffraction figure is acquired using said portion (805; 806) of the matrix photodetector (86).

6. Method according to any one of claims 1 to 4, wherein:
- the distance between the first transparent window (712) and the second transparent window (762) is variable in time, with the first transparent window and the second transparent window defining two planes parallel to each other, and with the immersible probe which comprises means (702, 703) for moving the second transparent window relative to the first transparent window; and
- the first transparent window is moved relative to the second transparent window; and
- said global diffraction figure is acquired with the use of the matrix photodetector (76).

7. Method according to claim 6, wherein the means (702, 703) for moving the second transparent window relative to the first transparent window include a rack driven in translation by a gear, and fixed relative to the second tight housing (761).

8. Method according to any one of claims 1 to 7, wherein the light source (71; 81) is spatially coherent.

9. Method according to any one of claims 1 to 8, wherein the immersible probe (92) is immersed in a liquid solution (91), said liquid solution (91) being located in a tank (95) of a bioreactor (90).

10. Method according to any one of claims 1 to 9, wherein the method then comprises the following steps:
- a global diffraction figure is acquired using the matrix photodetector (76; 86), the global diffraction figure comprising a plurality of elementary diffraction figures (20; 30) each associated with a cell, each elementary diffraction figure having a sequence of concentric closed curves; and
- a cell is classified as being in the living cells (68₁) or dead cell (68₂) category, depending on a light intensity on a central area (45; 46) of an elementary diffraction figure (20; 30) associated with said cell.
